# EUROPEAN PATENT SPECIFICATION

(11) **EP 3 347 345 B1**
(45) Date of publication and mention of the grant of the patent: **31.07.2019**
(21) Application number: 16774675.9
(22) Date of filing: 29.09.2016
(51) Int. Cl.: C07D 215/26

(54) **MIXED SOLVATE OF (R)-5-[2-(5,6-DIETHYLINDAN-2-YLAMINO)-1-HYDROXYETHYL]-8-HYDROXY-1H-QUINOLIN-2-ONE L-TARTRATE**
GEMISCHTES SOLVAT VON (R)-5-[2-(5,6-DIETHYLINDAN-2-YLAMINO)-1-HYDROXYETHYL]-8-HYDROXY-1H-CHINOLIN-2-ON-L-TARTRAT
SOLVATE MIXTE DE (R)-5-[2-(5,6-DIÉTHYLINDAN-2-YLAMINO)-1-HYDROXYÉTHYL]-8-HYDROXY-1H-QUINOLIN-2-ONE L-TARTRATE

(30) Priority: 29.09.2015 EP 15187430
(43) Date of publication of application: 18.07.2018
(73) Proprietor: INKE, S.A., 08755 Castellbisbal, Barcelona (ES)
(72) Inventor: CAPDEVILA URBANEJA, Enric, 08970 Sant Joan Despí - Barcelona (ES); HUGUET CLOTET, Juan, 08970 Sant Joan Despí - Barcelona (ES); DALMASES BARJOAN, Pere, 08970 Sant Joan Despí - Barcelona (ES)
(74) Representative: ABG Intellectual Property, S.L.
(86) International application number: PCT/EP2016/073355
(87) International publication number: WO 2017/055506

(56) References cited:
- WO-A1-2008/000839
- WO-A1-2014/044566
- WO-A1-2014/154841

## Description

### FIELD OF THE INVENTION

The present invention provides an improved process for the manufacture of indacaterol or a pharmaceutically acceptable salt thereof, preferably indacaterol maleate, in high chemical and enantiomeric purity and high yield, via a mixed solvate of (R)-5-[2-(5,6-diethylindan-2-ylamino)-1-hydroxyethyl]-8-hydroxy-1H-quinolin-2-one L-tartrate salt. The invention also relates to the mixed solvate of (R)-5-[2-(5,6-diethylindan-2-ylamino)-1-hydroxyethyl]-8-hydroxy-1H-quinolin-2-one L-tartrate salt, to a process for preparing the mixed solvate and to its use for the manufacture indacaterol or a pharmaceutically acceptable salt thereof.

### BACKGROUND OF THE INVENTION

(R)-5-[2-(5,6-diethylindan-2-ylamino)-1-hydroxyethyl]-8-hydroxy-1H-quinolin-2-one, the compound of formula (I), known as indacaterol, is an ultra-long acting β₂-adrenoreceptor agonist, commercialized as its maleate salt under the brand name of Onbrez Breezhaler®, for the once daily treatment of chronic obstructive pulmonary disease (COPD).

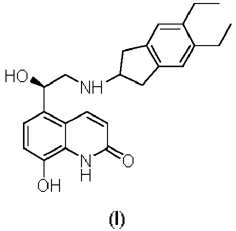

WO00/75114 and WO2004/076422 disclose the preparation of indacaterol, as shown in Scheme 1. According to said scheme, the chiral epoxide (R)-8-benzyloxy-5-oxiranyl-1H-quinolin-2-one reacts with 2-amino-5,6-diethylindane to provide a complex crude, containing about 20% of compounds (VII) and (VIII) as impurities. According to WO2004/076422, key intermediate (VI) is isolated from the complex crude as the benzoate salt and recrystallized. Then, purified benzoate salt is hydrogenated and further reacted with maleic acid to provide indacaterol maleate in an overall yield of 43%. This route implies several drawbacks. Firstly, a high amount of impurities are produced in order to provide intermediate (VI). Secondly, impurities of tartrate and benzoate salts may easily be present in the final indacaterol maleate since indacaterol is purified by means of both tartrate and benzoate salts. In addition, enantiomeric purities are not disclosed.

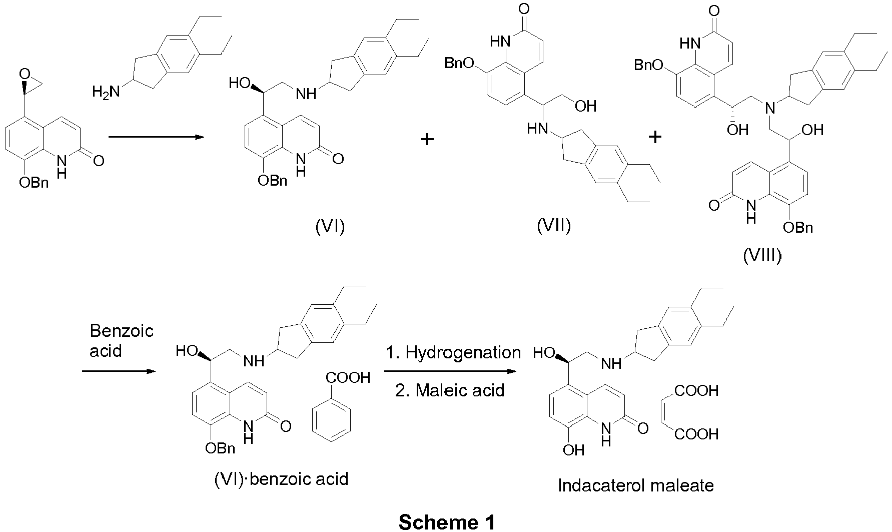

WO2005/123684 discloses a process for preparing the chiral epoxide in high enantiomeric purity using metal based chiral agents and a base, and its use for preparing indacaterol maleate through the same method described in previous WO2004/76422. Enantiomeric purities are not disclosed.

WO2014/044566 discloses the preparation of indacaterol maleate through the process depicted in Scheme 2. Intermediate (IX) is reacted with 2-amino-5,6-diethylindane to provide intermediate (X), which is reacted with hydrochloric acid, neutralized and further reacted with L-tartaric acid to provide intermediate (VI)·L-tartaric acid, which is further purified. Then, after a neutralization step, the obtained intermediate (VI) is hydrogenated and reacted with maleic acid to provide indacaterol maleate. It has been found that further impurities are generated due to the hydrogenation step, so the indacaterol maleate thus obtained does not have the purity required to be used in the manufacture of pharmaceutical compositions. Also, enantiomeric purity of indacaterol maleate is not disclosed.

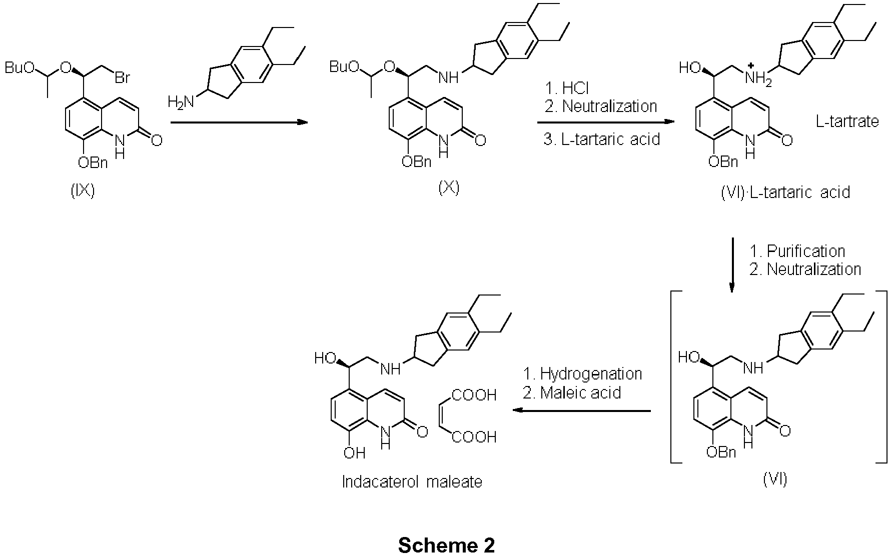

WO2014/154841 discloses the synthesis of indacaterol maletate via the hemi-L-tartrate salt of intermediate (VI). The hemi-L-tartrate salt is neutralized to provide intermediate (VI), which is hydrogenated and further reacted with maleic acid to provide indacaterol maleate in 98.6% of enantiomeric purity. As already explained in the previous paragraph, it has been found that further impurities are generated during the step of hydrogenation of intermediate (VI).

In the process described in WO2014/154841, the last step for obtaining indacaterol always involves the removal of the protecting group on the phenolic hydroxyl and this step introduces undesired impurities in the resulting indacaterol. This is clearly visible in example 10 of WO2014/154841 wherein the conversion of the hemi-L-tartrate salt of (R)-8-(benzyloxy)-5-[2-(5,6-diethylindan-2-ylamino)-1-hydroxyethyl]-1H-quinolin-2-one (having an enantiomeric purity of 99.87% and a chemical purity of 99.62%) into the maleate salt of ((*R*)-5-[2-(5,6-diethylindan-2-ylamino)-1-hydroxyethyl]-8-hydroxy-1H-quinolin-2-one), the maleate salt of indacaterol, results in a product having a purity of 99.7% but an enantiomeric purity of only 98.6% after the hydrogenation step.

In addition to the known maleate salt of indacaterol, WO2008/000839 discloses the preparation of several salts of indacaterol suitable for oral inhaled formulations. As an example, the indacaterol hydrogen L-tartrate salt, wherein the molar ratio of indacaterol to L-tartaric acid is 1:1, is prepared from indacaterol and L-tartaric acid. Purities are not disclosed.

Under the regulatory point of view, all chiral bioactive drug molecules must be isolated and tested for the efficacy and safety and have to be as pure as possible, containing a single pure enantiomer. So not only single enantiomer drug can have more selective pharmacologic profile and/or improved therapeutic indices, but also can differ in toxicity and/or adverse side effects. Specially, enantiomeric impurities of chiral bioactive drugs having high potency activity, such as indacaterol, can also have high potency activity, toxicity and/or adverse side effects. Thus, a synthetic route which can provide indacaterol or a pharmaceutically acceptable salt thereof, such as indacaterol maleate, having low amount of impurities is also desired.

Therefore, there is a need in developing feasible methods for the manufacture of indacaterol or a pharmaceutically acceptable salt thereof in high purity and high yield.

### BRIEF DESCRIPTION OF THE INVENTION

The present inventors have surprisingly found a mixed solvate of ethanol and water of the indacaterol L-tartrate salt useful for preparing indacaterol or a pharmaceutically acceptable salt thereof, preferably the commercial maleate salt, in high chemical and enantiomeric purity in a reproducible manner and in high yield. The use of said mixed solvate enables the obtention of indacaterol or a pharmaceutically acceptable salt thereof, preferably the commercial maleate salt, avoiding the generation of further impurities, particularly those having a very similar structure and chemical properties to the final product.

Thus, a first aspect of the present invention provides a mixed solvate of ethanol and water of indacaterol L-tartrate salt, compound of formula (II), wherein the molar ratio of indacaterol to L-tartaric acid is 2:1, wherein the molar ratio of indacaterol to ethanol is 2:1 and wherein the molar ratio of indacaterol to water is 2:1.

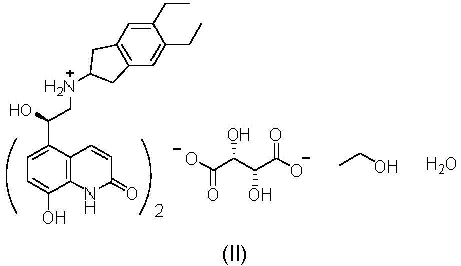

A second aspect of the present invention provides an improved process for preparing a pharmaceutically acceptable salt of indacaterol, preferably indacaterol maleate, comprising the use of the mixed solvate as defined in the first aspect.

A third aspect of the present invention provides an improved process for preparing indacaterol, comprising the use of the mixed solvate as defined in the first aspect.

A fourth aspect of the present invention relates to processes for preparing the mixed solvate according to the first aspect of the invention.

A fifth aspect of the present invention relates to the use of mixed solvate as defined in the first aspect for preparing indacaterol or a pharmaceutically acceptable salt thereof, preferably indacaterol maleate.

### BRIEF DESCRIPTION OF THE DRAWINGS

Examples of the invention are illustrated with the following drawings:
**Figure 1** shows the PXRD analysis of the mixed solvate of ethanol and water of indacaterol L-tartrate salt prepared as in example 3.
**Figure 2** shows the DSC-TGA analysis of the mixed solvate of ethanol and water of indacaterol L-tartrate salt prepared as in example 3.

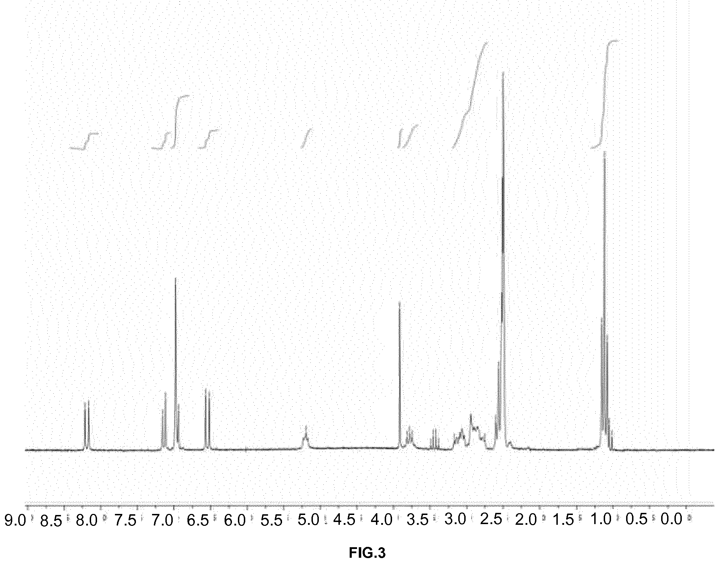

**Figure 3** shows the ¹H-RMN analysis of the mixed solvate of ethanol and water indacaterol L-tartrate salt prepared as in example 3, wherein the ratio of indacaterol to L-tartaric acid is 2:1.
**Figure 4** shows the PXRD analysis of the solid form, referred to as form II, of (R)-8-(benzyloxy)-5-[2-(5,6-diethylindan-2-ylamino)-1-hydroxyethyl]-1H-quinolin-2-one L-tartrate (2:1) salt, as prepared in example 2.
**Figure 5** shows the PXRD analysis of the solid form, referred to as form II, of (R)-8-(benzyloxy)-5-[2-(5,6-diethylindan-2-ylamino)-1-hydroxyethyl]-1H-quinolin-2-one hydrochloride (1:1) salt as prepared in example 1.

### DEFINITIONS

When describing the compounds and methods of the invention, the following terms have the following meanings, unless otherwise indicated.

As used herein the term **organic solvent** refers to an organic molecule capable of dissolving another substance (i.e., the solute). Organic solvents may be liquids at room temperature. Examples of organic solvents that may be used for the present invention include, but are not limited to: hydrocarbon solvents (e.g., n-pentane, n-hexane, n-heptane, n-octane, cyclohexane, methylcyclohexane, decahydronaphthalene, etc.) which also includes aromatic hydrocarbon solvents (e.g., benzene, toluene, o-xylene, m-xylene, and p-xylene), halogenated hydrocarbon solvents (e.g., carbon tetrachloride, 1,2-dichloroethane, dichloromethane, chloroform, etc.), ester solvents (e.g., ethyl formate, methyl acetate, ethyl acetate, isopropyl acetate, butyl acetate, isobutyl acetate, ethyl malonate, etc.), ketone solvents (e.g., acetone, methyl ethyl ketone or 2-butanone, methyl isobutyl ketone, cyclohexanone, cyclopentanone, 3-pentanone, etc.), ether solvents (e.g., diethyl ether, dipropyl ether, diphenyl ether, isopropyl ether, tert-butyl methyl ether, tetrahydrofuran, 1,4-dioxane, etc.), amine solvents (e.g., propyl amine, diethylamine, triethylamine, aniline, pyridine), alcohol solvents (e.g., methanol, ethanol, isopropanol, 1-propanol, 2-methyl-1-propanol, 1-butanol, 2-butanol, 1-pentanol, 3-methyl-1-butanol, tert-butanol, 1-octanol, benzyl alcohol, phenol, trifluoroethanol, glycerol, ethylene glycol, propylene glycol, m-cresol, etc.), acid solvents (e.g., acetic acid, hexanoic acid, etc.), nitrobenzene, N,N-dimethylformamide, N,N,-dimethylacetamide, dimethylsulfoxide, N-methyl-2-pyrrolidone, acetonitrile, silicone solvents (e.g., silicone oils, polysiloxanes, cyclosilicones). In some embodiments, the organic solvent may be formed by the combination of two or more organic solvents.

The term **polar solvent** as used herein means a solvent having a dielectric constant of at least 3, said dielectric constant being the ratio of the electrical capacity of a capacitor filled with the solvent to the electrical capacity of the evacuated capacitor at 20-25 °C. The values of dielectric constant of solvents are disclosed in Vogel's Textbook of Practical Organic Chemistry 5th Edition, Appendix 5. Examples of polar solvents are dichloromethane, tetrahydrofuran, ester solvents (e.g., ethyl formate, methyl acetate, ethyl acetate, ethyl malonate, etc.), ketone solvents (e.g., acetone, methyl ethyl ketone or 2-butanone, cyclohexanone, cyclopentanone, 3-pentanone, etc.), amine solvents (e.g., propyl amine, diethylamine, triethylamine, aniline, pyridine), alcohol solvents (e.g., methanol, ethanol, isopropanol, 1-propanol, 1-butanol, 1-octanol, benzyl alcohol, phenol, trifluoroethanol, glycerol, ethylene glycol, propylene glycol, m-cresol, etc.), acid solvents (e.g., acetic acid, hexanoic acid, etc.), nitrobenzene, N,N-dimethylformamide, N,N,-dimethylacetamide, dimethyl sulfoxide, N-methyl-2-pyrrolidone, acetonitrile, and silicone solvents (e.g., silicone oils, polysiloxanes, cyclosilicones).

The term **alcohol** refers to a hydrocarbon derivative in which one or more hydrogen atoms have been replaced by an -OH group, known as hydroxyl group. Suitable alcohols for the present invention include linear, cyclic or branched C₁-C₆ alkyl alcohols and any mixtures thereof. It also includes commercially available alcohols. Examples of alcohols are methanol, ethanol, isopropanol, 1-propanol, 1-butanol, 1-pentanol, 3-methyl-1-butanol, tert-butanol, 1-octanol, benzyl alcohol and phenol.

The term **room temperature** in the context of the present invention means that the temperature is between 10 °C and 40 °C, preferably between 15 °C and 30 °C, more preferably between 20 °C and 25 °C.

The term **conventional purification techniques** as used herein refers to the process wherein a product can be obtained in high purity, which can be carried out on an industrial scale such as solvent extraction, filtration, distillation, slurring, washing, phase separation, evaporation, centrifugation, isolation or crystallization.

As used herein, the term, **solvent extraction** refers to the process of separating components of a mixture by using a solvent which possesses greater affinity for one component, and may therefore separate said one component from at least a second component which is less miscible than said one component with said solvent.

The term **filtration** refers to the act of removing solid particles greater than a predetermined size from a feed comprising a mixture of solid particles and liquid. The expression filtrate refers to the mixture less the solid particles removed by the filtration process. It will be appreciated that this mixture may contain solid particles smaller than the predetermined particle size. The expression "filter cake" refers to residual solid material remaining on a feed side of a filtration element.

As used herein, the term **slurring** refers to any process which employs a solvent to wash or disperse a crude product.

As used herein, the term **washing** refers to the process of purifying a solid mass (e.g., crystals) by passing a liquid over and/or through the solid mass, as to remove soluble matter. The process includes passing a solvent, such as distilled water, over and/or through a precipitate obtained from filtering, decanting, or a combination thereof. For example, in one embodiment of the invention, washing includes contacting solids with solvent or solvent mixture, vigorously stirring (e.g., for two hours), and filtering. The solvent can be water, can be an aqueous solvent system, or can be an organic solvent system. Additionally, the washing can be carried out with the solvent having any suitable temperature. For example, the washing can be carried out with the solvent having a temperature between about 0 °C and about 100 °C.

The term **phase separation** refers to a solution or mixture having at least two physically distinct regions.

The term **crystallization** refers to any method known to a person skilled in the art such as crystallization from single solvent or combination of solvents by dissolving the compound, optionally at elevated temperature, and precipitating or allowing to precipitate (crystallize) the compound by cooling the solution, allowing it to cool, removing the solvent from the solution, or any combination thereof. It further includes methods such as those comprising the use of a solvent (for dissolving the compound)/antisolvent (for precipitating the compound) or precipitation.
The term **solvate** refers to solid molecular compounds that have incorporated the crystallizing solvent molecule in their lattice. When the solvent incorporated in the solvate is water, is called hydrate. All solvates are formed with stoichiometric or non-stoichiometric proportions between the compound and the solvent of crystallization. Solvates can exhibit polymorphism.

The term **purification** and/or **purifying** as used herein refer to the process wherein a purified drug substance can be obtained, measured by HPLC preferably having a purity greater than 80%, more preferably greater than 85%, more preferably greater than 90%, more preferably greater than 95%, more preferably greater than 99%, more preferably greater than 99.5%, even more preferably greater than 99.9%. The term "industrial purification" refers to purifications, which can be carried out on an industrial scale such as solvent extraction, filtration, slurring, washing, phase separation, evaporation, centrifugation or crystallization.

The term indacaterol or pharmaceutically acceptable salt thereof in **high chemical purity** refers to a purity obtained by HPLC of at least 99%, preferably of at least 99.5%, most preferably of at least 99.9%, even more preferably of at least 99.95%, even most preferably of at least 99.99%.

The term indacaterol or pharmaceutically acceptable salt thereof in **high enantiomeric purity** refers to an enantiomeric purity of the desired R-configuration measured by chiral HPLC of at least 99.90%, more preferably of at least 99.95%, even more preferably of at least 99.99%, even more preferably of 100.00%.

The term **pharmaceutically acceptable salt** according to the invention refers to a salt prepared from a base or their respective conjugated acids, which is acceptable for administration to a patient, such as a mammal. Such salts can be derived from pharmaceutically acceptable inorganic or organic acids, which include, but are not limited to, hydrochloric, hydrobromic, sulfuric, phosphoric, nitric, methansulfonic, ethansulfonic, benzenesulfonic, p-toluenesulfonic, camphorsulfonic, 1,5 -naphthalene disulfonic formic, acetic, benzoic, malonic, malic, citric, fumaric, gluconic, glutamic, lactic, maleic, mandelic, mucic, pantothenic, succinic, xinafoic (1-hydroxy-2-naphthoic acid) and cinnamic. Preferably, the pharmaceutically acceptable acid is maleic acid. The pKa is a measurement of the strength of an acid. The lower the pKa, the stronger the acidity. The higher the pKa, the weaker the acid. In this application the term pKa value refer to the pKa in water as determined at room temperature and atmospheric pressure.

### DETAILED DESCRIPTION OF THE INVENTION

### Mixed solvate of indacaterol L-tartrate salt

The inventors have surprisingly found a mixed solvate of ethanol and water of indacaterol L-tartrate salt which is useful for preparing indacaterol or pharmaceutically acceptable salt thereof, preferably its maleate salt, in high chemical and enantiomeric purity in a reproducible manner and high yield.

Thus, a first aspect of the present invention provides a mixed solvate of ethanol and water of indacaterol L-tartrate salt, the compound of formula (II), wherein the mixed solvate of ethanol and water of indacaterol L-tartrate salt is characterized by having a molar ratio of indacaterol, L-tartaric acid, ethanol and water of 2:1:1:1

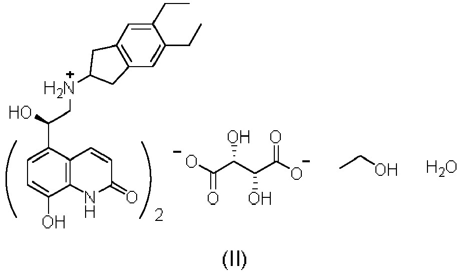

In a further embodiment of the first aspect, the mixed solvate of indacaterol L-tartrate salt is in crystalline solid form. Preferably, the crystalline solid form of the mixed solvate of indacaterol L-tartrate salt is characterized by a powder X-ray diffraction (PXRD) pattern having characteristics peaks at approximately 4.9, 14.9 and 24.2 ± 0.2 degrees two theta (i.e. Bragg's angle); measured by CuKα₁ radiation (λ = 1.54056 Å).

The term approximately in the context of the present invention means in the context of X-ray diffraction measurements that there is an uncertainty in the measurements of the degrees two theta of ± 0.2 (expressed in degrees two theta).

The mixed solvate of indacaterol L-tartrate salt is further characterized in that the powder X-ray diffraction pattern comprises the additional characteristic peaks at approximately 11.4, 19.1 and 23.2 ± 0.2 degrees two theta; measured by CuKα₁ radiation (λ = 1.54056 Å).

In another embodiment of the first aspect, the mixed solvate of indacaterol L-tartrate salt further provides a powder X-ray diffraction pattern; measured by CuKα₁ radiation (λ = 1.54056 Å), characterized by the interplanar distance d values shown below.

| Angle 2θ (°) (±0.2) | d value (Å) |
|---|---|
| 4.9 | 17.93 |
| 11.4 | 7.75 |
| 14.9 | 5.94 |
| 19.1 | 4.64 |
| 23.2 | 3.82 |
| 24.2 | 3.68 |

In a further embodiment, the mixed solvate of indacaterol L-tartrate salt provides a powder X-ray diffraction pattern; measured by CuKα₁ radiation (λ = 1.54056 Å), further characterized by the interplanar distance d and relative intensity (in percentage) at approximately the values shown below.

| Angle 2θ (°) (±0.2) | d value (Å) | Rel. Int. [%] |
|---|---|---|
| 4.9 | 17.93 | 100 |
| 11.4 | 7.75 | 31.4 |
| 14.9 | 5.94 | 28.9 |
| 19.1 | 4.64 | 38.7 |
| 23.2 | 3.82 | 29.8 |
| 24.2 | 3.68 | 26.5 |

The term approximately in the context of the present invention means in this context of PXRD intensity measurements that there is an uncertainty in the measurements of the relative intensities. It is known to the person skilled in the art that the uncertainty of the relative intensities depends strongly on the measurement conditions. The relative intensity values can e.g. vary by 30%.

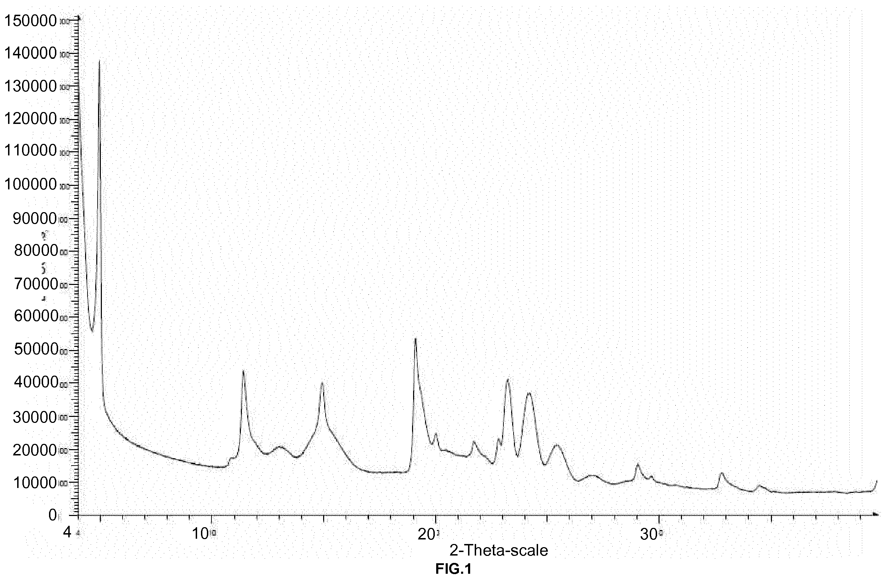

In a further embodiment of the first aspect, the invention provides a mixed solvate of indacaterol L-tartrate salt characterized in that it provides a PXRD pattern; measured by CuKα₁ radiation (λ = 1.54056 Å), substantially in accordance with Figure 1.

In a further embodiment of the first aspect, the mixed solvate of indacaterol L-tartrate salt is characterized by a DSC thermogram showing an endothermic peak with an onset at 175-177 °C.

In a further embodiment of the first aspect, the mixed solvate of indacaterol L-tartrate salt is further characterized by a DSC thermogram showing an endothermic peak with an onset at 98-100 °C.

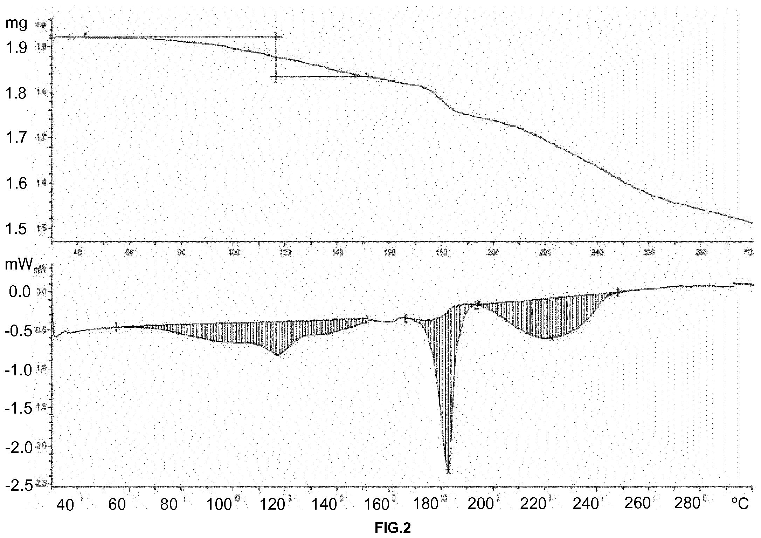

In a further embodiment of the first aspect, the mixed solvate of indacaterol L-tartrate salt is characterized in that it provides a DSC-TGA substantially in accordance with Figure 2.

In another embodiment of the first aspect, the mixed solvate of ethanol and water of indacaterol L-tartrate salt can be obtained wet, thus having ethanol and water being adsorbed on the solvate but not forming part of the crystal structure. In this sense, the ethanol and water molecules are thus admixed but not bonded within the mixed solvate lattice. The mixed solvate of ethanol and water of the first aspect is stable even if certain advantageous amounts of ethanol and water still remain admixed with the mixed solvate of the first aspect. Generally, the amount of non-binding ethanol in the mixed solvate of the first aspect can vary from 4% to 8% by weight based on the weight of the mixed solvate and the non-binding water in the mixed solvate of the first aspect can vary from 1.5% to 3% by weight based on the weight of the mixed solvate.

In a further embodiment, the invention encompasses the new mixed solvate of ethanol and water of indacaterol L-tartrate salt, in pure form or when admixed with other materials, for example, other polymorphs, solvates or remaining reaction solvents or side products.

The mixed solvate of indacaterol L-tartrate salt according to the first aspect is obtained in high chemical and enantiomeric purity and good stability. In addition, the mixed solvate according to the first aspect provides indacaterol or salts thereof, as indacaterol maleate, in high chemical and enantiomeric purity and high yield in a reproducible manner.

Particularly, the inventors of the present invention have realized that the prior art processes disclosed in documents WO2004/076422, WO2014/044566 and WO 2014/154841, provided indacaterol maleate having at least two significant active impurities, the (S)-enantiomeric impurity of formula (III) and an over-reduced impurity of formula (IV).

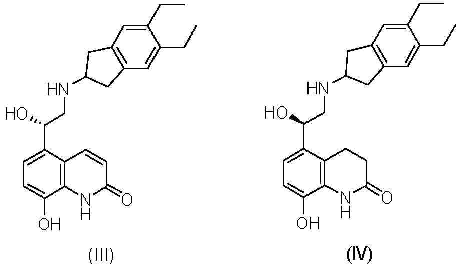

According to literature, see the article "Bioorg. Med. Chem. Lett., 2012, pages 6280-6285", the over-reduced impurity of formula (IV), which is defined as the 3,4-dihydroquinolinone derivate, can potentially cause systemic side effects. In fact, an in vivo study revealed that the associated systemic β2-adrenoreceptor effects, as measured by increases in heart rate, were greater and more persistent for compound (IV) when compared to indacaterol of formula (I). In addition, impurity of formula (IV) has a very similar chemical structure to indacaterol and thus very similar chemical properties being very difficult to separate from the final product indacaterol or salts thereof, such as indacaterol maleate, by standard purification and isolation techniques.

Advantageously, the use of the mixed solvate as defined in the first aspect is very useful in the preparation of indacaterol or a pharmaceutically acceptable salt thereof, such as indacaterol maleate, as it provides indacaterol and salts thereof having low concentrations of these undesired highly active impurities of formula (III) and (IV) in a reproducible manner.

Thus, the indacaterol or a pharmaceutically acceptable salt thereof, such as indacaterol maleate, prepared by the process of the present invention is suitable for preparing pharmaceutical compositions having very low amount of impurities. Particularly, the pharmaceutical compositions comprising indacaterol or a pharmaceutically acceptable salt thereof, such as indacaterol maleate, prepared by the process of the present invention have very low amount of impurities of formula (III) and formula (IV).

As explained above, the indacaterol or a pharmaceutically acceptable salt thereof, preferably indacaterol maleate, prepared by the process of the present invention are obtained in high enantiomeric purity, having a concentration of the (S)-enantiomer impurity of formula (III) below 0.1% measured by HPLC. More preferably, the concentration of the (S)-enantiomer impurity in the indacaterol or a pharmaceutically acceptable salt thereof, preferably indacaterol maleate, is below 0.05% measured by HPLC. Even more preferably, the concentration of the (S)-enantiomer impurity in the indacaterol or a pharmaceutically acceptable salt thereof, preferably indacaterol maleate, is below 0.03% measured by HPLC. Most preferably, the indacaterol provided is indacaterol maleate.

In addition, the indacaterol or a pharmaceutically acceptable salt thereof, preferably indacaterol maleate, prepared by the process of the present invention are obtained in high chemical purity, having low concentration of impurity of formula (IV) is below 0.3% measured by HPLC. More preferably, the concentration of the impurity of formula (IV) in the indacaterol or a pharmaceutically acceptable salt thereof, preferably indacaterol maleate, is below 0.1% measured by HPLC. Even more preferably, the concentration of the impurity of formula (IV) in the indacaterol or a pharmaceutically acceptable salt thereof, preferably indacaterol maleate, is below 0.05% measured by HPLC. Most preferably, the indacaterol provided is indacaterol maleate.

Also, the indacaterol or a pharmaceutically acceptable salt thereof, such as indacaterol maleate, prepared by the process of the present invention contains low concentration of total chemical impurities. Preferably, the concentration of total chemical impurities in the indacaterol or a pharmaceutically acceptable salt thereof, preferably indacaterol maleate, is below 0.3% measured by HPLC. More preferably, the concentration of the total chemical impurities in the indacaterol or a pharmaceutically acceptable salt thereof, preferably indacaterol maleate, is below0.1% measured by HPLC.

### Process for the manufacture of a pharmaceutically acceptable salt of indacaterol comprising the mixed solvate of indacaterol L-tartrate salt

Thus, a second aspect of the present invention provides a process for preparing a pharmaceutically acceptable salt of indacaterol, comprising at least the steps of:
a) reacting the mixed solvate of indacaterol L-tartrate salt of formula (II) as defined in the first aspect, with a pharmaceutically acceptable acid, in the presence of a solvent, and
b) isolating the product obtained in step a) to provide a pharmaceutically acceptable salt of indacaterol
or alternatively
a-i) reacting the mixed solvate of indacaterol L-tartrate salt of formula (II) as defined in the first aspect, with a base in the presence of a solvent to provide indacaterol, and
b-i) reacting the indacaterol obtained in step a-i) with a pharmaceutically acceptable acid, in the presence of a solvent, and
c-i) isolating the product obtained in step b-i) to provide a pharmaceutically acceptable salt of indacaterol

Suitable pharmaceutically acceptable acids of steps a) and/or b-i) are selected from hydrochloric, hydrobromic, sulfuric, methansulfonic, ethansulfonic, p-toluensulfonic, malonic, formic, acetic, malic, succinic, fumaric, glycolic, citric acid, maleic and 1-hydroxy-2-naphthoic acid. More preferably, the pharmaceutically acceptable acid of steps a) and/or b-i) is maleic acid.

In a particular embodiment of the second aspect, it provides a process for preparing indacaterol maleate, comprising at least the steps of:
a) reacting the mixed solvate of indacaterol L-tartrate salt as defined in the first aspect, with maleic acid in the presence of a solvent, and
b) isolating the product obtained in step a) to provide the maleate salt of indacaterol.

In a more particular embodiment of the second aspect, it provides a process for preparing indacaterol maleate, comprising at least the steps of:
a) reacting the mixed solvate of indacaterol L-tartrate salt as defined in the first aspect with maleic acid in the presence of a solvent, at a temperature from room temperature to the reflux temperature of the solvent to provide a solution and maintaining the reaction mixture until a precipitate is obtained, and
b) isolating the product obtained in step a) to provide the maleate salt of indacaterol.

In a more particular embodiment of the second aspect, it provides a process for preparing the indacaterol maleate, comprising at least the steps of:
a) reacting the mixed solvate of indacaterol L-tartrate salt as defined in the first aspect, with maleic acid in the presence of a solvent, wherein the solvent is a mixture of an alcohol and water in a ratio from 10:1 to 1:1 (v/v), at a temperature from room temperature to the reflux temperature of the solvent to provide a solution and maintaining the reaction mixture until a precipitate is obtained, and
b) isolating the product obtained in step i) to provide the maleate salt of indacaterol.

In a more preferred embodiment of the second aspect, it provides a process for preparing the maleate salt of indacaterol, comprising at least the steps of:
a) reacting the mixed solvate of indacaterol L-tartrate salt as defined in the first aspect, with maleic acid in the presence of a solvent, wherein the solvent is a mixture of ethanol and water in a ratio from 10:1 to 1:1 (v/v), at reflux temperature of the solvent to provide a solution and maintaining the reaction mixture until a precipitate is obtained, and
b) isolating the product obtained in step a) to provide the maleate salt of indacaterol.

In a more preferred embodiment of the second aspect, it provides a process for preparing the maleate salt of indacaterol, comprising at least the steps of:
a) reacting the mixed solvate of indacaterol L-tartrate salt as defined in the first aspect, with maleic acid in the presence of a solvent, wherein the solvent is a mixture of ethanol and water in a ratio from 10:1 to 5:1 (v/v), at the reflux temperature of the solvent to provide a solution and maintaining the reaction mixture until a precipitate is obtained, and
b) isolating the product obtained in step a) to provide the maleate salt of indacaterol.

Suitable solvents used in steps a) and/or b-i) are those in which the pharmaceutically acceptable salt of indacaterol obtained has a low solubility, wherein the pharmaceutically acceptable salt of indacaterol has a solubility range from 10 mg/mL to 33 mg/mL. Suitable solvents used in steps a) and/or b-i) comprises an organic solvent, water or mixtures thereof. Preferably, the solvent of steps a), a-i) and/or b-i) comprises a mixture of an organic solvent and water. Preferably, the organic solvent is a polar solvent. Preferably, the solvent of steps a), a-i) and/or b-i) comprises a mixture of a polar solvent and water. Preferably, the polar solvents are alcohols, ester and ketone solvents. More preferably, the solvent of steps a), a-i) and/or b-i) comprises a mixture of an alcohol and water. Suitable alcohols are linear or branched C1-C6 alcohols, such as methanol, ethanol, 1-propanol, isopropanol and mixtures thereof. Preferably, the solvent of step a) comprises a mixture of an alcohol and water in a ratio from 10:1 to 1:1 (v/v). More preferably, the solvent of step a) comprises a mixture of an alcohol and water in a ratio from 10:1 to 5:1 (v/v) as the purity of the pharmaceutically acceptable salt of indacaterol thus obtained is improved.

Advantageously, the use of the mixture of ethanol and water in a ratio from 10:1 to 1:1 (v/v), preferably in a ratio from 10:1 to 5:1 (v/v), at reflux temperature of the solvent increases the chemical and enantiomeric purity and the yield of the maleate salt of indacaterol thus obtained.

Suitable solvents used in step a-i) are those in which the indacaterol obtained is soluble, wherein the solubility of indacaterol in the solvent ranges from 33 mg/mL to 100 mg/mL. Suitable solvents used in step a-i) are organic solvents. Preferably, the organic solvents are polar solvents. Suitable polar solvents are halogenated hydrocarbon solvents, alcohols, esters, ketones, ether solvents and mixtures thereof. Suitable alcohols are linear or branched C₁-C₆ alcohols, such as methanol, ethanol, 1-propanol and isopropanol. Suitable halogenated hydrocarbon solvents are dichloromethane and chloroform. Suitable ester solvents are methyl acetate, ethyl acetate, isopropyl acetate, butyl acetate and isobutyl acetate. Suitable ketone solvents are acetone, methyl ethyl ketone and methyl isobutyl ketone. Suitable ether solvents are diethyl ether, dipropyl ether, diphenyl ether, isopropyl ether, tert-butyl methyl ether, tetrahydrofuran and 1,4-dioxane.

Preferably, the pharmaceutically acceptable salt of indacaterol prepared by the process of the second aspect of the invention may be further purified. More preferably, the pharmaceutically acceptable salt of indacaterol, wherein the pharmaceutically acceptable acid is maleic acid to provide indacaterol maleate may be purified by recrystallization.

The third aspect of the present invention also provides a process for preparing indacaterol, comprising at least the steps of:
i. reacting the mixed solvate of indacaterol L-tartrate salt as defined in the first aspect, with a base in the presence of a solvent to provide indacaterol, and
ii. isolating the product obtained in step i)

Suitable bases may be inorganic bases selected from hydroxides, carbonates and bicarbonates of calcium, sodium, magnesium, potassium, lithium and caesium and mixtures thereof. The amount of base used may be at least in a molar ratio of 1:1 of base to the mixed solvate of indacaterol L-tartrate salt. Suitable solvents may be those as defined in the second aspect.

Suitable solvent used in step i) comprises an organic solvent, water or mixtures thereof. Preferably, the solvent of step i) comprises a mixture of an organic solvent and water. Preferably, the organic solvent is a polar solvent. Preferably, the solvent of step i) comprises a mixture of a polar solvent and water. Suitable polar solvents are alcohols, ester and ketone solvents. More preferably, the solvent of step i) comprises a mixture of an alcohol and water. Suitable alcohols are linear or branched C₁-C₆ alcohols, such as methanol, ethanol, 1-propanol, isopropanol and mixtures thereof. Preferably, the solvent of step i) comprises a mixture of an alcohol and water in a ratio from 10:1 to 1:1 (v/v). More preferably, the solvent of step i) comprises a mixture of an alcohol and water in a ratio from 10:1 to and 5:1 (v/v) as the purity of the pharmaceutically acceptable salt of indacaterol thus obtained is improved.

### Process for preparing the mixed solvate of indacaterol L-tartrate salt

Thus, a fourth aspect of the present invention relates to a process for preparing the mixed solvate as defined in the first aspect of the present invention, comprising the steps of:
i. removing the protecting group R of compound of formula (V) by hydrogenation in the presence of a catalyst and an organic solvent, 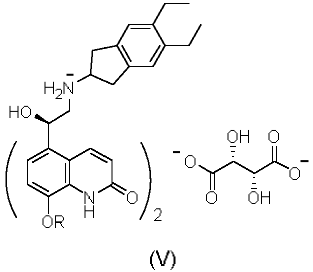 wherein the protecting group R of compound of formula (V) is an hydroxyl protecting group
ii. removing the solvent of step i), and
iii. dissolving the crude product obtained in step ii) in a solvent mixture comprising ethanol and water and leaving to rest until a precipitate is obtained, and
iv. isolating the precipitate obtained in step iii) to provide the mixed solvate of indacaterol L-tartrate salt of the first aspect

Suitable hydroxyl protecting groups R of compound of formula (V) are those which can be deprotected by hydrogenation such as 4-methylbenzyl, 2-methylbenzyl, 4-methoxybenzyl, 2-methoxybenzyl, 2,4-dimethoxybenzyl, 3,4-dimethoxybenzyl, 2,6-dimethoxybenzyl, 4-nitrobenzyl, 2-nitrobenzyl, 2,4-dinitrobenzyl, 4-chlorobenzyl and 2-chlorobenzyl. Preferably, the hydroxyl protecting group R is benzyl and 4-methoxybenzyl. More preferably, the hydroxyl protecting group R is benzyl.
The hydrogenation step i) is conducted in the presence of a catalyst and an organic solvent. Suitable catalysts are selected from Pd, Pt, Rh, Ru, Ni, Fe, Zn and Ir catalyst. Preferably, suitable catalyst are selected form palladium (0) (Pd(0)), palladium hydroxide (Pd(OH)₂), palladium on activated carbon (Pd/C), palladium on alumina, palladium on carbon powder, platinum, platinum on activated carbon and Raney™ nickel. A combination of catalysts may also be used. Most preferably, the catalyst is palladium on activated carbon and palladium hydroxide (Pd(OH)₂). The amount of catalyst is not critical and may be equal or less than 10% by weight to the amount of compound of formula (V). Moreover, the hydrogenation takes place at a hydrogen pressure range of from 0.5 to 10 atm. Most preferably, the hydrogen pressure is from 0.5 to 2 atm since fewer impurities are generated.

The organic solvent used in step i) may be polar and non-polar solvents. Preferably the organic solvent used in the deprotection step i) is a polar solvent selected from alcohols, esters, ethers, C₁-C₆ linear, branched or cyclic carboxylic acids such as acetic acid and mixtures thereof. Examples of alcohols include C₁-C₆ linear, branched or cyclic alcohols, such as methanol, ethanol, isopropanol, or mixtures thereof. Examples of esters include ethyl acetate, isopropyl acetate and mixtures thereof. Examples of ethers include tetrahydrofuran, dioxane and mixtures thereof. Preferably, the solvent used in step i) is a polar solvent selected from alcohols, carboxylic acids such as acetic acid and mixtures thereof. More preferably, the solvent is acetic acid.

The reaction temperature used in step i) according to the fourth aspect may take place over a range of temperature from room temperature to the reflux temperature of the selected solvent. Preferably, the reaction is carried out at room temperature so that the mixed solvate is obtained in high purity since fewer impurities are generated.

In a particular embodiment, the hydrogenation step i) is carried out in the presence of a catalyst as defined above, in the presence of a polar solvent selected from alcohols, carboxylic acids such as acetic acid and mixtures thereof, at room temperature and at hydrogen pressure selected from 0.5 to 10 atm.

In a particular embodiment, the solvent is partially removed in step ii) by distillation until a wet solid is obtained. The distillation is preferably carried out at a temperature below 50°C and under vacuum since the percentage of impurities generated is reduced.

The crucial point of the process according to the fourth aspect is step iii), wherein the crude product of step ii) is dissolved in a solvent mixture comprising ethanol and water, preferably in a ratio from 10:1 to 1:1 (v/v). More preferably, the ratio of ethanol and water is from 5:1 to 1:1 (v/v). The temperature of step iii) may be from room temperature to the reflux temperature of the solvent mixture to provide a solution. Preferably, the temperature of step iii) is the reflux temperature of the solvent mixture.

In a preferred embodiment, the step iii) of the fourth aspect of the invention is carried out in the presence of the mixture of ethanol and water in a molar ratio from 5:1 to 1:1 (v/v) at reflux temperature of the solvent mixture to provide a solution and leaving to rest until a precipitate is obtained, and isolating the precipitate obtained in step iii) to provide the mixed solvate of the first aspect in high yield and in high purity.

In one embodiment, the solution obtained in step iii) may be seeded with the mixed solvate as defined in the first aspect.

In one embodiment, the mixed solvate according to the first and fourth aspect may be dried. Drying may be carried out at atmospheric pressure or under reduced pressure and at room temperature or at temperatures below 50 °C. Preferably, the mixed solvate according to the first and fourth aspect can be dried at under reduced pressure and at temperatures below 50 °C.

Advantageously, the process of the fourth aspect of the present invention contributes to the preparation of the mixed solvate of indacaterol L-tartrate salt of the first aspect in high chemical and enantiomeric purity. Particularly, the over-reduced impurity of formula (IV) and the enantiomeric impurity of formula (III) are significantly reduced specially to the crystallization and isolation of the mixed solvate according to the first aspect.

Thus, the mixed solvate of indacaterol L-tartrate salt according to the first aspect prepared by the process of the fourth aspect is obtained in high chemical purity, having a concentration of impurity of formula (IV) below 0.3% measured by HPLC. Preferably, the concentration of the impurity of formula (IV) is below 0.1% measured by HPLC. More preferably, the concentration of the impurity of formula (IV) is below 0.05% measured by HPLC. Even more preferably, the concentration of the impurity of formula (IV) is below 0.03% measured by HPLC.

Thus, the mixed solvate of indacaterol L-tartrate salt according to the first aspect prepared by the process of the fourth aspect is advantageously obtained in high enantiomeric purity of at least 99.90%, preferably of at least 99.95%, more preferably of at least 99.99% measured by HPLC, having a concentration of the (S)-enantiomer impurity of formula (III) below 0.1% measured by HPLC. More preferably, the concentration of the (S)-enantiomer impurity is below 0.05% measured by HPLC. Even more preferably, the concentration of the (S)-enantiomer impurity is below 0.03% measured by HPLC.

Advantageously, the use of the same solvent mixture of ethanol and water in a ratio from 10:1 to 1:1 (v/v) either in the crystallization step of the mixed solvate as defined in the first aspect and in the preparation of the pharmaceutically acceptable salt of indacaterol, preferably its maleate salt, significantly simplifies the operability, costs and quality controls, such as the residual solvent content, at industrial scale.

Moreover, the indacaterol or salt thereof, as indacaterol maleate, as prepared via the mixed solvate of indacaterol L-tartrate salt is advantageously obtained in high enantiomeric purity measured by HPLC of at least 99.90%, preferably of at least 99.95%, even more preferably of at least 99.99%, having a concentration of the (S)-enantiomer impurity of formula (III) below 0.1% measured by HPLC. More preferably, the concentration of the (S)-enantiomer impurity is below 0.05% measured by HPLC. Even more preferably, the concentration of the (S)-enantiomer impurity is below 0.03% measured by HPLC.

According to the fourth aspect, the compound of formula (V) used in step i) contains two molecules of protected indacaterol, wherein the protecting group R of compound of formula (V) is an hydroxyl protecting group, and 1 molecule of L-tartaric acid and can be present in any crystalline form, solvate (including hydrates) or amorphous form.

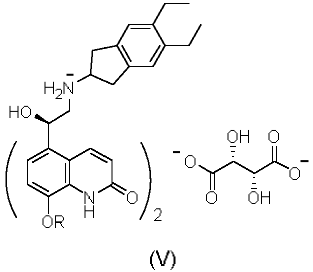

In a particular embodiment, the protecting group R of compound of formula (V) is benzyl, the compound is (R)-8-(benzyloxy)-5-[2-(5,6-diethylindan-2-ylamino)-1-hydroxyethyl]-1H-quinolin-2-one L-tartrate (2:1) salt as shown below.

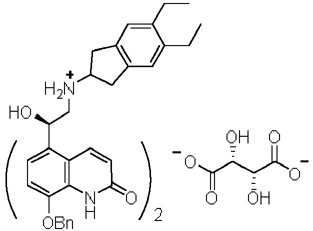

In another particular embodiment, the compound (R)-8-(benzyloxy)-5-[2-(5,6-diethylindan)-2-ylamino-1-hydroxyethyl]-1H-quinolin-2-one L-tartrate (2:1) salt is in crystalline solid form and can be prepared according to example 6 of document WO2014/154841.

In a preferred embodiment, (R)-8-(benzyloxy)-5-[2-(5,6-diethylindan-2-ylamino)-1-hydroxyethyl]-1H-quinolin-2-one L-tartrate (2:1) salt is in crystalline form, also referred to as form II, characterized by at least one of the following:
a) An IR having the characteristics bands at approximately 3380, 3329, 3047, 2963, 1664, 1585, 1339, 1058, 834 and 743 cm⁻¹; or
b) A DSC thermogram showing a sharp endothermic peak with an onset at 209-211 °C and a peak at 212-214 °C.

In a further embodiment, the crystalline solid form of (R)-8-(benzyloxy)-5-[2-(5,6-diethylindan-2-ylamino)-1-hydroxyethyl]-1H-quinolin-2-one L-tartrate (2:1) salt, also referred to as form II, is characterized by a powder X-ray diffraction pattern having characteristic peaks degrees two theta and the interplanar distance d values; measured with a CuKα source (λ = 1.541874 Å), shown below.

| Angle 2θ (°) (±0.2) | d value (Å) |
|---|---|
| 4.5 | 19.51 |
| 10.7 | 8.22 |
| 12.0 | 7.33 |
| 12.4 | 7.13 |
| 13.5 | 6.55 |
| 14.2 | 6.22 |
| 14.8 | 5.97 |
| 17.3 | 5.11 |
| 18.0 | 4.91 |
| 19.0 | 4.66 |
| 19.2 | 4.61 |
| 25.8 | 3.44 |
| 27.2 | 3.27 |

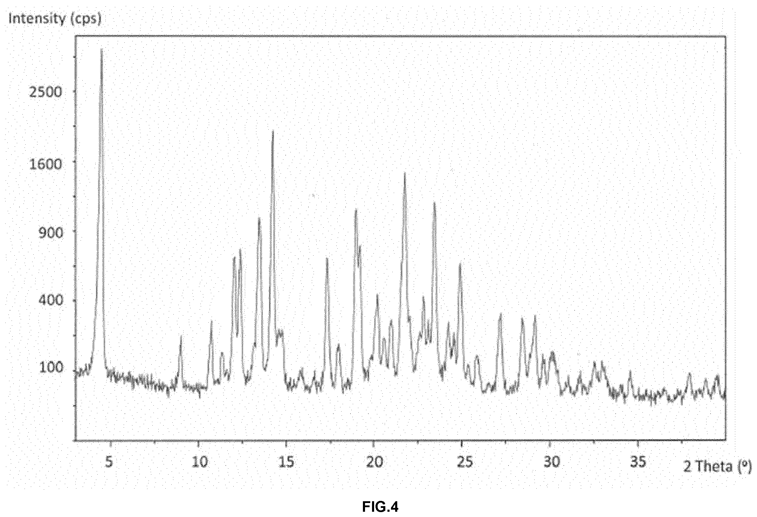

In a further embodiment, the crystalline solid form of (R)-8-(benzyloxy)-5-[2-(5,6-diethylindan-2-ylamino)-1-hydroxyethyl]-1H-quinolin-2-one L-tartrate (2:1) salt, also referred to as form II, is further characterized in that it provides a PXRD pattern substantially in accordance to Figure 4.

Advantageously, the L-tartrate (2:1) salt of (R)-8-(benzyloxy)-5-[2-(5,6-diethylindan-2-ylamino)-1-hydroxyethyl]-1H-quinolin-2-one in crystalline solid form, referred to as form II, is useful for the manufacture of the mixed solvate of indacaterol as defined in the first aspect since the purity of the mixed solvate of indacaterol is improved.

In another embodiment of the fourth aspect, the (R)-8-(benzyloxy)-5-[2-(5,6-diethyl-indan-2-ylamino)-1-hydroxyethyl]-1H-quinolin-2-one L-tartrate (2:1) salt, can be obtained from the hydrochloride salt of (R)-8-(benzyloxy)-5-[2-(5,6-diethylindan-2-ylamino)-1-hydroxyethyl]-1H-quinolin-2-one, which can be prepared either from example 3 of document WO2004/076422 or example 2 of document WO2014/154841.

In a more preferred embodiment, the process for preparing (R)-8-(benzyloxy)-5-[2-(5,6-diethylindan-2-ylamino)-1-hydroxyethyl]-1H-quinolin-2-one L-tartrate (2:1) salt, comprises the steps of:
i. reacting the compound of formula (VI)·HCl, the (R)-8-(benzyloxy)-5-[2-(5,6-diethylindan-2-ylamino)-1-hydroxyethyl]-1H-quinolin-2-one hydrochloride salt, 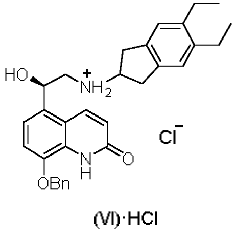 wherein the molar ratio of protected indacaterol and hydrochloric acid is 1:1, with a base in the presence of an organic solvent and water to provide (R)-8-(benzyloxy)-5-[2-(5,6-diethylindan-2-ylamino)-1-hydroxyethyl]-1H-quinolin-2-one, and
ii. reacting the product of step i) with L-tartaric acid in the presence of an organic solvent to provide the L-tartrate (2:1) salt of (R)-8-(benzyloxy)-5-[2-(5,6-diethylindan-2-ylamino)-1-hydroxyethyl]-1H-quinolin-2-one, and
iii. isolating the product obtained in step ii)

Suitable bases used in step i) may be inorganic bases selected from hydroxides, carbonates and bicarbonates of calcium, sodium, magnesium, potassium, lithium and caesium.

Suitable organic solvents used in step i) may be polar solvents such as dichloromethane, tetrahydrofuran, ester solvents such as ethyl formate, methyl acetate, ethyl acetate, ethyl malonate and ketone solvents such as acetone, methyl ethyl ketone or 2-butanone, cyclopentanone, cyclohexanone, 3-pentanone. Preferably, the solvent used in step i) is dichloromethane.

The amount of L-tartaric acid used in step ii) to provide the L-tartrate (2:1) salt of (R)-8-(benzyloxy)-5-[2-(5,6-diethylindan-2-ylamino)-1-hydroxyethyl]-1H-quinolin-2-one, is preferably in a molar ratio of 1:2 of L-tartaric acid to compound (R)-8-(benzyloxy)-5-[2-(5,6-diethylindan-2-ylamino)-1-hydroxyethyl]-1H-quinolin-2-one.

Suitable organic solvents used in step ii) may be polar solvents such as alcohols, ketones and mixtures thereof. Suitable alcohols include methanol, ethanol and isopropanol. Suitable ketones include acetone, methyl ethyl ketone or 2-butanone, cyclopentanone, cyclohexanone, 3-pentanone.

In a more preferred embodiment, the (R)-8-(benzyloxy)-5-[2-(5,6-diethylindan-2-ylamino)-1-hydroxyethyl]-1H-quinolin-2-one L-tartrate (2:1) salt in crystalline solid form II is obtained using a mixture of methanol and acetone as solvent in step ii).

In a particular embodiment, the (R)-8-(benzyloxy)-5-[2-(5,6-diethylindan-2-ylamino)-1-hydroxyethyl]-1H-quinolin-2-one hydrochloride (1:1) salt of step i) is in crystalline solid form.

Preferably, the crystalline solid form of the (R)-8-(benzyloxy)-5-[2-(5,6-diethylindan-2-ylamino)-1-hydroxyethyl]-1H-quinolin-2-one hydrochloride (1:1) salt, referred to as form II, is characterized by at least one of the following:
a) A powder X-ray diffraction (PXRD) pattern having characteristics peaks at approximately 4.0, 8.1, 14.3 and 16.3 ± 0.2 degrees two theta (i.e. Bragg's angle); values measured with CuKα source (λ = 1.541874 Å), or
b) A DSC thermogram showing an endothermic peak with an onset at 251-253 °C.

In a further embodiment, the crystalline solid form of (R)-8-(benzyloxy)-5-[2-(5,6-diethylindan-2-ylamino)-1-hydroxyethyl]-1H-quinolin-2-one hydrochloride (1:1) salt, referred to as form II, is further characterized in that the powder X-ray diffraction pattern further comprises the following peaks at approximately 4.0, 5.6, 8.1, 10.0, 14.3, 16.3 and 24.1 ± 0.2 degrees two theta; values measured with CuKα source (λ = 1.541874Å).

In a further embodiment, the crystalline solid form of (R)-8-(benzyloxy)-5-[2-(5,6-diethylindan-2-ylamino)-1-hydroxyethyl]-1H-quinolin-2-one hydrochloride (1:1) salt, referred to as form II, is further characterized by a powder X-ray diffraction pattern having the following characteristic degrees two theta peaks and the interplanar distance d values; measured with CuKα source (λ = 1.541874Å), shown below.

| Angle 2θ (°) (±0.2) | d value (Å) |
|---|---|
| 4.0 | 21.74 |
| 5.6 | 15.52 |
| 8.1 | 10.83 |
| 10.0 | 8.81 |
| 14.3 | 6.17 |
| 16.3 | 5.41 |
| 24.1 | 3.68 |

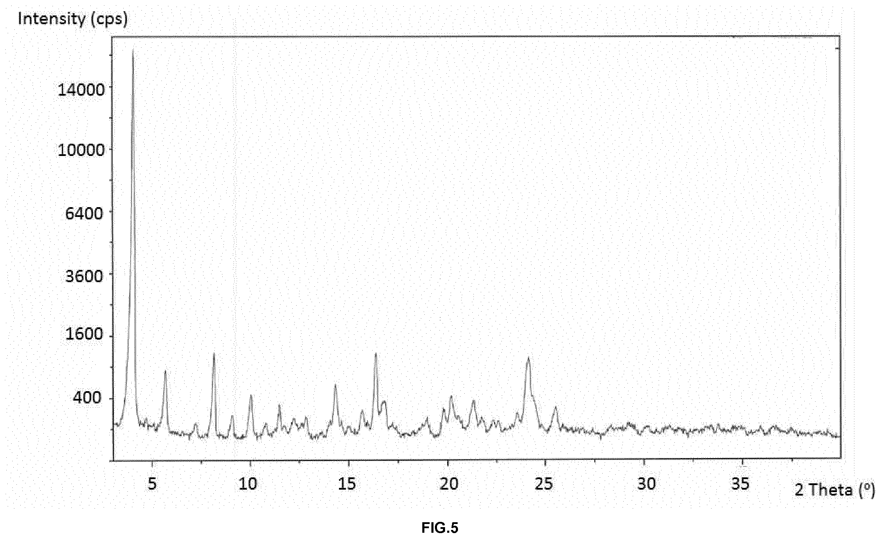

In a further embodiment, the invention provides the crystalline solid form of (R)-8-(benzyloxy)-5-[2-(5,6-diethylindan-2-ylamino)-1-hydroxyethyl]-1H-quinolin-2-one hydrochloride (1:1) salt, referred to as form II, characterized in that it provides a PXRD pattern substantially in accordance to Figure 5.

Advantageously, the form II of hydrochloride (1:1) salt of (R)-8-(benzyloxy)-5-[2-(5,6-diethyl-indan-2-ylamino)-1-hydroxyethyl]-1H-quinolin-2-one is very useful for preparing the L-tartrate (2:1) salt of (R)-8-(benzyloxy)-5-[2-(5,6-diethyl)indan-2-ylamino-1-hydroxyethyl]-1H-quinolin-2-one in high purity and high yield.

A fifth aspect of the present invention relates to the use of mixed solvate of indacaterol L-tartrate salt as defined in the first aspect for preparing indacaterol or a pharmaceutically acceptable salt thereof, preferably indacaterol maleate, in high chemical and enantiomeric purity and high yield.

In the following, the present invention is further illustrated by examples. They should in no case be interpreted as a limitation of the scope of the invention as defined in the claims.

### EXPERIMENTAL

### General methods

### Proton Nuclear Magnetic Resonance (¹H NMR)

Proton nuclear magnetic resonance analyses were recorded in a deuterated solvent in a Varian Gemini 200 Fourier-Transform (FT) NMR spectrometer and chemical shifts are given in part per million (ppm) downfield from tetramethylsilane as internal standard. The coupling constants are given in Hz. Spectra were acquired dissolving 5-10 mg of sample in 0.7 mL of deuterated solvent.

### Elemental Analysis (EA)

Elemental analyses were recorded in a LECO CHNS-932 elemental analyzer. Equipped with and IR absorption detector and a thermal conductivity TC detector.

### Differential Scanning Calorimetry (DSC)

DSC analyses were recorded in a Mettler Toledo DSC822e calorimeter. Experimental conditions: 40 µL aluminium crucibles; atmosphere of dry nitrogen at 50 mL/min flow rate; heating rate of 10 °C/min between 30 and 300°C. Data collection and evaluation was done with software STARe.

### Infrared spectrometry (IR)

Infrared spectrometry analyses were recorded in a Perkin Elmer FT-IR spectrum One appliance using a Perkin Elmer ATR accessory.

### Powder X-Ray Diffraction (PXRD)

The PXRD pattern of Figure 1 was acquired on a D8 Advance Series 2Theta/Theta powder diffraction system using CuKα₁-radiation (λ = 1.54056 Å) in transmission geometry. The system is equipped with a VANTEC-1 single photon counting PSD, a Germanium monochromator, a ninety positions auto changer sample stage, fixed divergence slits and radial soller. The sample was measured in a 60 minute scans in a range from 4° to 40° in 2θ. Programs used: Data collection with DIFFRAC plus XRD Commander V.2.5.1 and evaluation with EVA V.12.0.

The PXRD patterns of figures 4 and 5 were acquired using a Philips X'Pert powder diffractometer, equipped with a CuKα source (λ = 1.541874 Å; Kα₂/α₁ = 0.5), and a proportional detector, operating at 50 kV and 40 mA. Each sample was scanned between 3° and 40° in 2θ, with a step size of 0.03° and a scan rate of 1 s/step. Data collection carried out with High Score Plus.

### High Performance Liquid Chromatography (HPLC)

HPLC was used to determine either the enantiomeric purity of indacaterol maleate and of indacaterol L-tartrate of the present invention.

HP 1090M HPLC machine was used with the following conditions:
Column: Chiralpak-AD-H 0.25 m x 4.6 mm, 5 µm
Column temperature: 35 °C
Injection volume: 10 µL
Solvent: methanol
Mobile phase: n-hexane + ethanol + methanol 84:8.8:0.5 v/v/v
Flow rate: 1.3 mL/min
Detection: UV at 260 nm
Run time: 13 min
Relative retention time:
   (R)-indacaterol maleate: 1
   (S)-indacaterol maleate: 1.58

### Examples

### Example 1. (R)-8-(benzyloxy)-5-[2-(5,6-diethylindan-2-ylamino)-1-hydroxyethyl]-1H-quinolin-2-one hydrochloride (1:1) salt

A mixture of 8-benzyloxy-5-(1R)-2-bromo-1-[tert-butyldimethylsilyloxy]-ethyl-quinolin-2-1H-one (1.0 Kg), 2-amino-5,6-diethylindane hydrochloride (0.51 Kg), NaHCO₃ (0.43 Kg) in DMSO (1.0 L) were heated to 65 °C under inert atmosphere. The suspension obtained was heated to 125 °C and stirred for 6 h. The brownish solution obtained was cooled to 80-85 °C and toluene (5.0 L) was added. Then, the brownish solution was cooled to 50-55 °C and water (5.0 L) was added. The resulting mixture was cooled to 20-25 °C and stirred for 15 min. Phases were separated and the aqueous phase was extracted with toluene (2.0 L). Organic phases were joined and washed with water. The organic phase was concentrated at 35 °C under reduced pressure. The brownish oil obtained was dissolved in ethanol (4.0 L) and 3.0 L of solvent mixture were distilled at 70 °C under reduced pressure. A solution of ethanol (1.4 L) and hydrochloric acid 37% (0.44 L) was added at a temperature lower than 70 °C. The solution obtained was stirred at 70-72 °C for 24 h and then cooled to 0-5 °C for 2 h and stirred at this temperature range for 1 h. The solid obtained was filtered, washed with a cool mixture of ethanol and hydrochloric acid 37% (21:4), washed with cold isopropanol and dried at 40 °C.
Yield: 0.76 Kg (71%)
Purity (HPLC): 99.53%. Enantiomeric purity (HPLC): 98.60%
DSC (10°C/min): Sharp endothermic peak with an onset at 251-253 °C.

### Example 2. (R)-8-(benzyloxy)-5-[2-(5,6-diethylindan-2-ylamino)-1-hydroxyethyl]-1H-quinolin-2-one L-tartrate (2:1) salt

(R)-8-(benzyloxy)-5-[2-(5,6-diethylindan-2-ylamino)-1-hydroxyethyl]-1H-quinolin-2-one hydrochloride (25 g) was stirred with dichloromethane (125 mL) and a solution of sodium carbonate (5.8 g) in water (125 mL) at 20- 25 °C for 15 min. Phases were separated and the aqueous phase was extracted with dichloromethane (50 mL). Organic phases were joined and washed with water (50 mL). The organic phase was concentrated at 35 °C under reduced pressure. The brownish oil obtained was dissolved in methanol (150 mL) and partially of the solvent was distilled (50 mL) at 35 °C under reduced pressure. Acetone (100 mL) was added and the resulting solution was seeded with the (R)-8-(benzyloxy)-5-[2-(5,6-diethylindan-2-ylamino)-1-hydroxyethyl]-1H-quinolin-2-one L-tartrate (2:1) salt. A solution of L-(+)-tartaric acid (7.3 g) in methanol (100 mL) was added for 30 min at 30 °C. The white suspension obtained was cooled down to 20-25 °C, stirred at for 1 h, filtered off, washed with a mixture of acetone and methanol (1:2) and dried at 40 °C for 4 hours.
Yield: 19.5 g (73%)
Chemical purity (HPLC): 99.66%. Enantiomeric purity (HPLC): 99.40%
DSC (10°C/min): Sharp endothermic peak with onset at 209-211 °C and a peak at 212-214 °C, due to melting and initial decomposition.

### Example 3. Preparation of mixed solvate of (R)-5-[-2-(5,6-diethylindan-2-ylamino)-1-hydroxyethyl]-8-hydroxy-1H-quinolin-2-one tartrate

A solution of (R)-8-(benzyloxy)-5-[2-(5,6-diethylindan-2-ylamino)-1-hydroxyethyl]-1H-quinolin-2-one L-tartrate (2:1) salt (25.0 g, 44.8 mmol) in acetic acid (125 mL) was stirred with Pd/C 5% (1.9 g) under a pressure of 1 atm of hydrogen at 20-25 °C for 5 h. The mixture was filtered over a pad of filter aid, which was rinsed with acetic acid. The filtrate was concentrated at 35 °C under reduced pressure until a thick white paste was obtained. Ethanol (100 mL) was added and the resulting mixture was stirred at 35 °C for a while and concentrated at 35 °C under reduced pressure. More ethanol (100 mL) was added and the resulting mixture was stirred at 35 °C for a while and concentrated at 35 °C under reduced pressure. A mixture of 125 mL of ethanol and water (5:2) was added to the thick white solid obtained and the resulting mixture was stirred and heated to 70-80 °C until a clear solution was obtained. The solution was cooled to 70 °C and seeded. The suspension obtained was cooled to 20-25 °C for 2 h 30 min, cooled to 0-5 °C and stirred at this temperature range for 1 h. The white solid was filtered, washed with a cold mixture of ethanol and water (5:2) and dried under vacuum at 20 °C for 5 hours.
Yield: 17.6 g (84%)
Chemical purity (HPLC): 98.95%. Enantiomeric purity (HPLC): 99.99%
DSC (10°C/min): First broad endothermic peak with onset at 99 °C (-85 J/g), due to evaporation of solvent (desolvation process), second sharp endothermic peak with onset at 176 °C (-71 J/g), due to melting and initial decomposition and third broad endothermic peak with onset at 197 °C (-80 J/g), due to decomposition.
TGA (10°C/min): Continuous weight loss observed. A first step from 30 °C and 160-170 °C due to evaporation of solvent (evaporation) and additional weight loss starting at 175 °C due to decomposition.
¹H NMR (200MHz, d6-DMSO): δ/ppm (TMS) 8.20 (d, J=10 Hz, 1H), 7.14 (d, J=8 Hz, 1H), 6.98 (s, 2H), 6.96 (d, J=8 Hz, 1H), 6.55 (d, J=10 Hz, 1H), 5.20 (m, 1H), 3.92 (s,1H), 3.82-3.78 (m, 1H), 3.44 (q, J=6 Hz, 0.4H, CH₃CH₂OH), 3.17-2.75 (m, 6H), 2.58 (q, J=8 Hz, 4H), 1.12 (t, J=8 Hz, 6H), 1.05 (t, J=6 Hz, 0.6H, CH₃CH₂OH).
Elemental analysis: Calculated: 64.91% C, 7.06% H, 5.61% N; Found: 64.51% C, 6.91% H, 5.90% N

### Example 4. Preparation of mixed solvate of (R)-5-[-2-(5,6-diethyl)indan-2-ylamino)-1-hydroxyethyl]-8-hydroxy-1H-quinolin-2-one tartrate

A solution of 9.5 g (17.0 mmol) of (R)-8-(benzyloxy)-5-[2-(5,6-diethylindan-2-ylamino)-1-hydroxyethyl]-1H-quinolin-2-one L-tartrate (2:1) salt, prepared in example 2 (Chemical purity (HPLC): 99.66%; Enantiomeric purity (HPLC): 99.40%), in acetic acid (48 mL) was stirred with Pd/C 5% (0.7 g) under a pressure of 1 atm of hydrogen at 20-25 °C for 5 h. The mixture was filtered over a pad of filter aid, which was rinsed with acetic acid. The filtrate was concentrated at 35 °C under reduced pressure until a thick white paste was obtained. Ethanol (40 mL) was added and the resulting suspension obtained was stirred at 35 °C for a while and concentrated at 35 °C under reduced pressure until a thick white solid was obtained. Chemical purity (HPLC): 98.94%; impurity of formula (IV): 0.3%. Enantiomeric purity (HPLC): 99.10%. Ethanol (40 mL) was added to the previous white solid and the white suspension obtained was stirred at 35 °C for a while and concentrated at 35 °C under reduced pressure. The white solid obtained was stirred with a mixture of 40 mL of ethanol and 20 mL of water and heated to 70-80 °C until a solution was obtained. The solution was cooled to 70 °C and seeded. The suspension obtained was stirred at 65-70 °C for 1 h, cooled to 20-25 °C for 3 h 30 min, cooled to 0-5 °C and stirred at this temperature range for 1 h. The white solid was filtered and washed with a cold mixture of ethanol and water (5:2).
Chemical purity (HPLC): 99.64%; impurity of formula (IV): 0.09%. Enantiomeric purity (HPLC): 100.00%

### Example 5. Preparation of (R)-5-[2-(5,6-diethylindan-2-ylamino)-1-hydroxyethyl]-8-hydroxy-1H-quinolin-2-one (2Z)-2-butenedioate, indacaterol maleate

(R)-5-[-2-(5,6-diethylindan-2-ylamino)-1-hydroxyethyl]-8-hydroxy-1H-quinolin-2-one L-tartrate (2:1) salt obtained in example 4 was suspended in 22 mL of a mixture of ethanol and water (100:15). Maleic acid (2.0 g, 17.0 mmol) was added and the suspension was heated to reflux. The obtained solution was cooled to 65-70 °C and seeded, cooled to 20-25 °C for 3 h 30 min, cooled to 0-5 °C and stirred at this temperature range for 1 h. The white solid was filtered, washed with a cold mixture of ethanol and water (100:15) and dried under vacuum at 40-45 °C.
Yield: 6.5 g (75%)
Chemical purity (HPLC): 99.87%; impurity of formula (IV): 0.04%. Enantiomeric purity (HPLC): 100.00%
DSC: Sharp endotherm with an onset at 207 °C.
PXRD analysis coincides with the solid form Qalpha described in document WO2008/025816.

## Claims

1. A mixed solvate of ethanol and water of indacaterol L-tartrate salt, compound of formula (II), wherein the molar ratio of indacaterol to L-tartaric acid is of 2:1, wherein the molar ratio of indacaterol to ethanol is 2:1 and wherein the molar ratio of indacaterol to water is of 2:1.

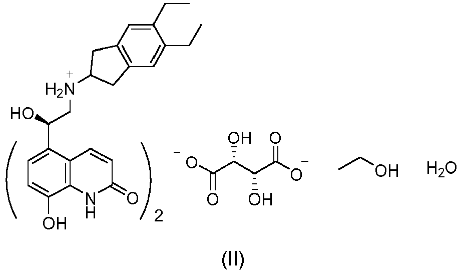

2. The mixed solvate according to claim 1, **characterized in that** it is in crystalline solid form.

3. The mixed solvate according to anyone of claims 1 and 2, wherein the crystalline solid form is **characterized by** a powder X-ray diffraction pattern (PXRD) having characteristic peaks at 4.9, 11.4, 14.9, 19.1, 23.2 and 24.2 ± 0.2 degrees two theta.

4. The mixed solvate according to anyone of claims 1 to 3, further **characterized by** a DSC thermogram showing a characteristic endothermic peak with an onset at 175-177 °C.

5. A process for preparing the mixed solvate of indacaterol L-tartrate salt according to anyone of claims 1 to 4, comprising the steps of:
i. removing the protecting group R of compound of formula (V) by hydrogenation in the presence of a catalyst and an organic solvent, 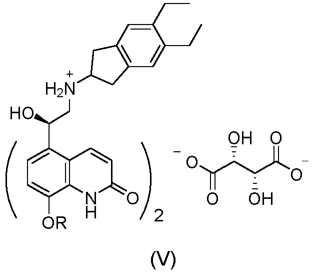 wherein the protecting group R of compound of formula (V) is a hydroxyl protecting group
ii. removing the solvent of step i), and
iii. dissolving the crude product obtained in step ii) in a solvent mixture comprising ethanol and water and leaving to rest until a precipitate is obtained, and
iv. isolating the solid obtained in step iii) to provide the mixed solvate of indacaterol L-tartrate salt

6. The process according to claim 5, wherein the solvent of step iii) is a mixture of ethanol and water in a ratio from 10:1 to 1:1 (v/v).

7. The process according to anyone of claims 5 to 6, wherein the protecting group R of compound of formula (V) of step i) is benzyl, the compound (R)-8-(benzyloxy)-5-[2-(5,6-diethylindan-2-ylamino)-1-hydroxyethyl]-1H-quinolin-2-one L-tartrate (2:1) salt, preferably in crystalline solid form.

8. The process according to anyone of claims 5 to 7, wherein the crystalline solid form, referred to as form II, is **characterized by** at least one of the following:
a) An IR having the characteristics bands at 3380, 3329, 3047, 2963, 1664, 1585, 1339, 1058, 834 and 743 cm⁻¹; or
b) A DSC thermogram showing an endothermic peak with an onset at 209-211 °C and a peak at 212-214 °C.

9. The process according to anyone of claims 5 to 8, wherein (R)-8-(benzyloxy)-5-[2-(5,6-diethylindan-2-ylamino)-1-hydroxyethyl]-1H-quinolin-2-one L-tartrate (2:1) salt, preferably in crystalline solid form II, is prepared by a process comprising the steps of:
i. reacting the compound of formula (VI)·HCl, (R)-8-(benzyloxy)-5-[2-(5,6-diethylindan-2-ylamino)-1-hydroxyethyl]-1H-quinolin-2-one hydrochloride, 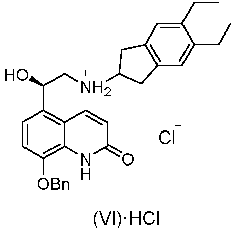 wherein the molar ratio of protected indacaterol and hydrochloric acid is 1:1, with a base in the presence of an organic solvent and water to provide the compound (R)-8-(benzyloxy)-5-[2-(5,6-diethyl-indan-2-ylamino)-1-hydroxyethyl]-1H-quinolin-2-one,
ii. reacting the product obtained in step i) with L-tartaric acid in the presence of an organic solvent to provide the (R)-8-(benzyloxy)-5-[2-(5,6-diethylindan-2-ylamino)-1-hydroxyethyl]-1H-quinolin-2-one L-tartrate (2:1) salt, and 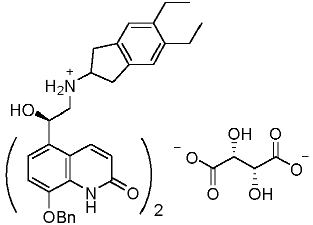
iii. isolating the product of step ii)

10. The process according to anyone of claims 5 to 9, wherein the organic solvent of step ii) is a mixture of methanol and acetone.

11. The mixed solvate according to anyone of claims 1 to 4 having less than 0.3% of impurity of formula (IV), as measured by HPLC.

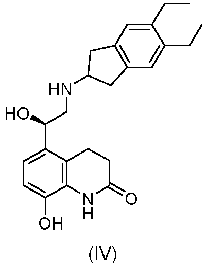

12. A process for the manufacture of a pharmaceutically acceptable salt of indacaterol, comprising at least the steps of:
a) reacting the mixed solvate of indacaterol L-tartrate salt as defined in claims 1 to 4, with a pharmaceutically acceptable acid, preferably maleic acid, in the presence of a solvent, and
b) isolating the product obtained in step a) to provide a pharmaceutically acceptable salt of indacaterol
or alternatively
a-i) reacting the mixed solvate of indacaterol L-tartrate salt as defined inclaims 1 to 4, with a base in the presence of a solvent to provide indacaterol
b-i) reacting the indacaterol obtained in step a-i) with a pharmaceutically acceptable acid, preferably maleic acid, in the presence of a solvent, and
c-i) isolating the product obtained in step b-i) to provide a pharmaceutically acceptable salt of indacaterol

13. The process according to anyone of claims 12 and 13, wherein the solvent used in steps a) and/or b-i) comprises a mixture of an alcohol and water, preferably a mixture of ethanol and water.

14. The process according to claim 13, wherein the ratio of the ethanol to water is from 10:1 to 1:1 (v/v).

15. The use of the mixed solvate according to anyone of claims 1 to 4 for the manufacture of a pharmaceutically acceptable salt of indacaterol, preferably indacaterol maleate.

## Patentansprüche

1. Gemischtes Solvat von Ethanol und Wasser des Indacaterol-L-tartrat-Salzes, der Verbindung der Formel (II), wobei das Stoffmengenverhältnis von Indacaterol zu L-Weinsäure 2:1 beträgt, wobei das Stoffmengenverhältnis von Indacaterol zu Ethanol 2:1 beträgt und wobei das Stoffmengenverhältnis von Indacaterol zu Wasser 2:1 beträgt:

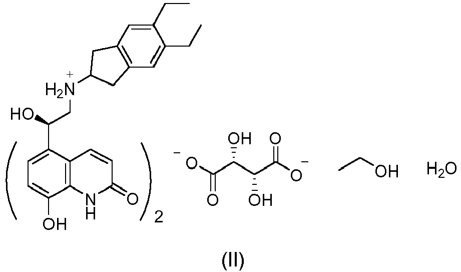

2. Gemischtes Solvat gemäß Anspruch 1, **dadurch gekennzeichnet, dass** es in kristalliner fester Form vorliegt.

3. Gemischtes Solvat gemäß einem der Ansprüche 1 und 2, wobei die kristalline feste Form durch ein Pulverröntgenbeugungsmuster (PXRD) gekennzeichnet ist, das charakteristische Reflexe bei 2 Theta = 4,9, 11,4, 14,9, 19,1, 23,2 und 24,2 ± 0,2 Grad aufweist.

4. Gemischtes Solvat gemäß einem der Ansprüche 1 bis 3, weiterhin **gekennzeichnet durch** ein DSC-Thermogramm, das einen charakteristischen endothermen Peak mit Einsetzen bei 175-177 °C zeigt.

5. Verfahren zur Herstellung des gemischten Solvats des Indacaterol-L-tartrat-Salzes gemäß einem der Ansprüche 1 bis 4, umfassend die Schritte:
i. Entfernen der Schutzgruppe R von der Verbindung der Formel (V) durch Hydrierung in Gegenwart eines Katalysators und eines organischen Lösungsmittels: 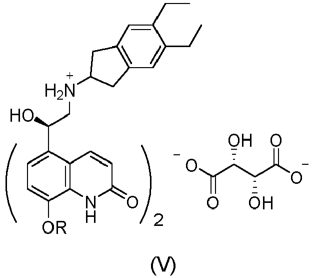 wobei die Schutzgruppe R der Verbindung der Formel (V) eine Hydroxy-Schutzgruppe ist;
ii. Entfernen des Lösungsmittels von Schritt i); und
iii. Auflösen des in Schritt ii) erhaltenen rohen Produkts in einem Lösungsmittelgemisch, das Ethanol und Wasser umfasst, und Ruhenlassen, bis ein Niederschlag erhalten wird; und
iv. Isolieren des in Schritt iii) erhaltenen Feststoffs, wobei man das gemischte Solvat des Indacaterol-L-tartrat-Salzes erhält.

6. Verfahren gemäß Anspruch 5, wobei das Lösungsmittel von Schritt iii) ein Gemisch von Ethanol und Wasser in einem Volumenverhältnis von 10:1 bis 1:1 ist.

7. Verfahren gemäß einem der Ansprüche 5 bis 6, wobei die Schutzgruppe R der Verbindung der Formel (V) von Schritt i) Benzyl ist und die Verbindung (R)-8-(Benzyloxy)-5-[2-(5,6-diethylindan-2-ylamino)-1-hydroxyethyl]-1H-chinolin-2-on-L-Tartrat(2:1)-Salz, vorzugsweise in kristalliner fester Form, ist.

8. Verfahren gemäß einem der Ansprüche 5 bis 7, wobei die kristalline feste Form, die als Form II bezeichnet wird, durch wenigstens eines der folgenden Merkmale gekennzeichnet ist:
a) ein IR mit den charakteristischen Banden bei 3380, 3329, 3047, 2963, 1664, 1585, 1339, 1058, 834 und 743 cm⁻¹; oder
b) ein DSC-Thermogramm, das einen endothermen Peak mit Einsetzen bei 209-211 °C und einen Peak bei 212-214 °C zeigt.

9. Verfahren gemäß einem der Ansprüche 5 bis 8, wobei (R)-8-(Benzyloxy)-5-[2-(5,6-diethylindan-2-ylamino)-1-hydroxyethyl]-1H-chinolin-2-on-L-Tartrat(2:1)-Salz, vorzugsweise in kristalliner fester Form II, durch ein Verfahren hergestellt wird, das die folgenden Schritte umfasst:
i. Umsetzen der Verbindung der Formel (VI) HCl, (R)-8-(Benzyloxy)-5-[2-(5,6-diethylindan-2-ylamino)-1-hydroxyethyl]-1H-chinolin-2-on-Hydrochlorid: 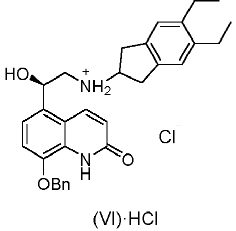 wobei das Stoffmengenverhältnis von geschütztem Indacaterol zu Chlorwasserstoffsäure 1:1 beträgt, mit einer Base in Gegenwart eines organischen Lösungsmittels und von Wasser, was die Verbindung (R)-8-(Benzyloxy)-5-[2-(5,6-diethylindan-2-ylamino)-1-hydroxyethyl]-1H-chinolin-2-on ergibt;
ii. Umsetzen des in Schritt i) erhaltenen Produkts mit L-Weinsäure in Gegenwart eines organischen Lösungsmittels, was das (R)-8-(Benzyloxy)-5-[2-(5,6-diethylindan-2-ylamino)-1-hydroxyethyl]-1H-chinolin-2-on-L-Tartrat(2:1)-Salz ergibt; und 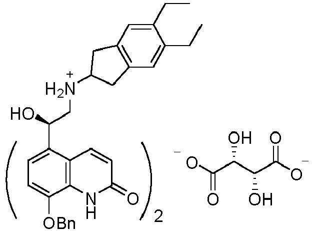
iii. Isolieren des Produkts von Schritt ii).

10. Verfahren gemäß einem der Ansprüche 5 bis 9, wobei das organische Lösungsmittel von Schritt ii) ein Gemisch von Methanol und Aceton ist.

11. Gemischtes Solvat gemäß einem der Ansprüche 1 bis 4, das gemäß einer Messung durch HPLC weniger als 0,3% Verunreinigung der Formel (IV) aufweist:

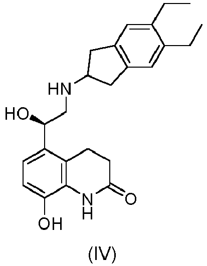

12. Verfahren zur Herstellung eines pharmazeutisch annehmbaren Salzes von Indacaterol, umfassend wenigstens die folgenden Schritte:
a) Umsetzen des gemischten Solvats des Indacaterol-L-tartrat-Salzes, wie es in den Ansprüchen 1 bis 4 definiert ist, mit einer pharmazeutisch annehmbaren Säure, vorzugsweise Maleinsäure, in Gegenwart eines Lösungsmittels; und
b) Isolieren des in Schritt a) erhaltenen Produkts, was ein pharmazeutisch annehmbares Salz von Indacaterol ergibt;
oder alternativ dazu
a-i) Umsetzen des gemischten Solvats des Indacaterol-L-tartrat-Salzes, wie es in den Ansprüchen 1 bis 4 definiert ist, mit einer Base in Gegenwart eines Lösungsmittels, was Indacaterol ergibt;
b-i) Umsetzen des in Schritt a-i) erhaltenen Indacaterols mit einer pharmazeutisch annehmbaren Säure, vorzugsweise Maleinsäure, in Gegenwart eines Lösungsmittels; und
c-i) Isolieren des in Schritt b-i) erhaltenen Produkts, was ein pharmazeutisch annehmbares Salz von Indacaterol ergibt.

13. Verfahren gemäß einem der Ansprüche 12 und 13, wobei das in den Schritten a) und/oder b-i) verwendete Lösungsmittel ein Gemisch eines Alkohols und Wasser, vorzugsweise ein Gemisch von Ethanol und Wasser, umfasst.

14. Verfahren gemäß Anspruch 13, wobei das Volumenverhältnis von Ethanol zu Wasser 10:1 bis 1:1 beträgt.

15. Verwendung des gemischten Solvats gemäß einem der Ansprüche 1 bis 4 zur Herstellung eines pharmazeutisch annehmbaren Salzes von Indacaterol, vorzugsweise Indacaterolmaleat.

## Revendications

1. Solvate mixte d'éthanol et d'eau de sel L-tartrate d'indacatérol, composé de la formule (II), où le rapport molaire de l'indacatérol à l'acide L-tartrique est de 2:1, où le rapport molaire de l'indacatérol à l'éthanol est de 2:1 et où le rapport molaire de l'indacatérol à l'eau est de 2:1.

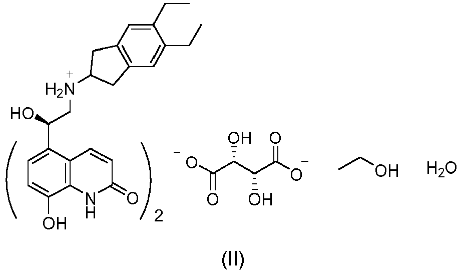

2. Solvate mixte selon la revendication 1, **caractérisé en ce qu'**il se présente sous forme solide cristalline.

3. Solvate mixte selon la revendication 1 ou 2, où la forme solide cristalline est **caractérisée par** un diagramme de diffraction des rayons X sur poudre (XRPD) présentant des pics caractéristiques à 4,9, 11,4, 14,9, 19,1, 23,2 et 24,2 ± 0,2 degrés deux thêta.

4. Solvate mixte selon l'une quelconque des revendications 1 à 3, **caractérisé en outre par** un thermogramme de calorimétrie différentielle à balayage présentant un pic endothermique caractéristique avec début à 175-177 °C.

5. Procédé de préparation du solvate mixte de sel L-tartrate d'indacatérol selon l'une quelconque des revendications 1 à 4, comprenant les étapes suivantes :
i. éliminer le groupe protecteur R du composé de formule (V) par hydrogénation en présence d'un catalyseur et d'un solvant organique, 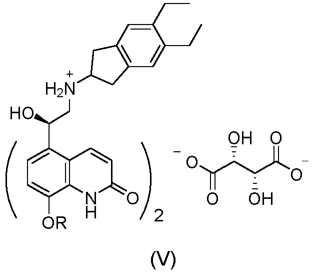 où le groupe protecteur R du composé de formule (V) est un groupe protecteur d'hydroxyle
ii. retirer le solvant de l'étape i), et
iii. dissoudre le produit brut obtenu à l'étape ii) dans un mélange de solvant comprenant de l'éthanol et de l'eau et laisser reposer jusqu'à l'obtention d'un précipité, et
iv. isoler le solide obtenu à l'étape iii) afin d'obtenir le solvate mixte de sel L-tartrate d'indacatérol

6. Procédé selon la revendication 5, où le solvant de l'étape iii) est un mélange d'éthanol et d'eau dans un rapport de 10:1 à 1:1 (v/v).

7. Procédé selon l'une quelconque des revendications 5 ou 6, où le groupe protecteur R du composé de formule (V) de l'étape i) est benzyle, le composé de sel L-tartrate (2:1) de (R)-8-(benzyloxy)-5-[2-(5,6-diéthylindan-2-ylamino)-1-hydroxyéthyl]-1H-quinolin-2-one, de préférence sous forme solide cristalline.

8. Procédé selon l'une quelconque des revendications 5 à 7, où la forme solide cristalline, désignée forme II, est **caractérisée par** au moins un parmi les suivants :
a) Un spectre IR présentant les bandes caractéristiques à 3380, 3329, 3047, 2963, 1664, 1585, 1339, 1058, 834 et 743 cm⁻¹ ; ou
b) Un thermogramme de calorimétrie différentielle à balayage présentant un pic endothermique avec début à 209-211 °C et un pic à 212-214 °C.

9. Procédé selon l'une quelconque des revendications 5 à 8, où le sel de L-tartrate (2:1) de (R)-8-(benzyloxy)-5-[2-(5,6-diéthylindan-2-ylamino)-1-hydroxyéthyl]-1H-quinolin-2-one, de préférence sous forme solide cristalline II, est préparé à l'aide d'un procédé comprenant les étapes suivantes :
i. mettre en réaction le composé de formule (VI)•HCl, hydrochlorure de (R)-8-(benzyloxy)-5-[2-(5,6-diéthylindan-2-ylamino)-1-hydroxyéthyl]-1H-quinolin-2-one, 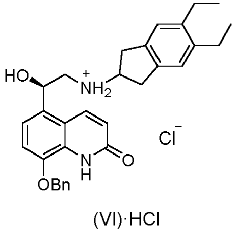 où le rapport molaire d'indacatérol protégé et d'acide chlorhydrique est 1:1, avec une base en présence d'un solvant organique et d'eau afin d'obtenir le composé (R)-8-(benzyloxy)-5-[2-(5,6-diéthylindan-2-ylamino)-1-hydroxyéthyl]-1H-quinolin-2-one,
ii. mettre en réaction le produit obtenu à l'étape i) avec de l'acide L-tartrique en présence d'un solvant organique afin d'obtenir le sel de L-tartrate (2:1) de (R)-8-(benzyloxy)-5-[2-(5,6-diéthylindan-2-ylamino)-1-hydroxyéthyl]-1H-quinolin-2-one, et 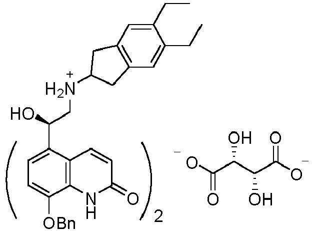
iii. isoler le produit de l'étape ii)

10. Procédé selon l'une quelconque des revendications 5 à 9, où le solvant organique de l'étape ii) est un mélange de méthanol et d'acétone.

11. Solvate mixte selon l'une quelconque des revendications 1 à 4 contenant moins de 0,3 % d'impuretés de formule (IV), tel que déterminé par la chromatographie en phase liquide de haute performance (HPLC).

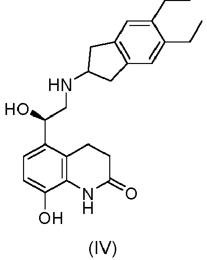

12. Procédé de fabrication d'un sel pharmaceutiquement acceptable d'indacatérol, comprenant au moins les étapes suivantes :
a) mettre en réaction le solvate mixte de sel L-tartrate d'indacatérol tel que défini selon les revendications 1 à 4, avec un acide pharmaceutiquement acceptable, de préférence de l'acide maléique, en présence d'un solvant, et
b) isoler le produit obtenu à l'étape a) afin d'obtenir un sel pharmaceutiquement acceptable d'indacatérol
ou alternativement
a-i) mettre en réaction le solvate mixte de sel L-tartrate d'indacatérol tel que défini selon les revendications 1 à 4, avec une base en présence d'un solvant afin d'obtenir de l'indacatérol
b-i) mettre en réaction l'indacatérol obtenu à l'étape a-i) avec un acide pharmaceutiquement acceptable, de préférence de l'acide maléique, en présence d'un solvant, et
c-i) isoler le produit obtenu à l'étape b-i) afin d'obtenir un sel pharmaceutiquement acceptable d'indacatérol

13. Procédé selon la revendication 12 ou 13, où le solvant utilisé aux étapes a) et/ou b-i) comprend un mélange d'un alcool et d'eau, de préférence un mélange d'éthanol et d'eau.

14. Procédé selon la revendication 13, où le rapport de l'éthanol à l'eau est de 10:1 à 1:1 (v/v).

15. Utilisation du solvate mixte selon l'une quelconque des revendications 1 à 4 pour la fabrication d'un sel pharmaceutiquement acceptable d'indacatérol, de préférence maléate d'indacatérol.
